# EUROPEAN PATENT APPLICATION

(11) **EP 4 094 722 A1**
(43) Date of publication of application: **30.11.2022**
(21) Application number: 21176205.9
(22) Date of filing: 27.05.2021
(51) Int. Cl.: A61C 19/06, A61C 17/00, A61C 17/22, A46B 15/00, G16H 10/60, G16H 30/40, G16H 40/63, G16H 50/20

(54) **GENERATING RECOMMENDED MOUTH CLEANING AND/OR TREATMENT CHARACTERISTICS**

(71) Applicant: Koninklijke Philips N.V., 5656 AG Eindhoven (NL)
(72) Inventor: RMAILE, Amir Hussein, 5656 AE Eindhoven (NL); HELAOUI, Rim, 5656 AE Eindhoven (NL); GERHARDT, Lutz Christian, 5656 AE Eindhoven (NL); DE RUYTER, Boris Emmanuel Rachmund, 5656 AE Eindhoven (NL); KOOIJMAN, Gerben, 5656 AE Eindhoven (NL)
(74) Representative: Philips Intellectual Property & Standards

(57) **Abstract**

An approach for generating recommended cleaning and/or treatment characteristics for different parts of a subject's mouth. Quality data about two or more first parts of the mouth is obtained. At least one cleaning and/or treatment characteristic for each of two or more second parts of the mouth are generated by (for each second part) processing the quality data of any overlapping first parts of the mouth.

## Description

### FIELD OF THE INVENTION

The present invention relates to the field of oral hygiene and, in particular, to the field of mouth cleaning and/or treatment.

### BACKGROUND OF THE INVENTION

In the field of dental or oral hygiene, a standard brushing time of 2 minutes is recommended for everyone worldwide. It has been identified that a brushing time of 2 minutes is usually sufficient to evenly brush teeth to an acceptable level.

Recent powered toothbrushes are adapted or programmed to follow this recommended brushing time, e.g. by providing feedback to a user when 2 minutes has elapsed since the brushing session began. Some toothbrushes are configured to distribute these 2 minutes over different segments (e.g. quadrants) of the user's mouth. For instance, it is known for a powered toothbrush to provide feedback to a user every 30 seconds, e.g. to indicate that the user should move the powered toothbrush to a different quadrant of their mouth.

There is an ongoing desire to improve oral hygiene habits and ensure that recommended mouth cleaning/treatment characteristics, such as a brushing time, are suited to a subject.

### SUMMARY OF THE INVENTION

The invention is defined by the claims.

According to examples in accordance with an aspect of the invention, there is provided a computer-implemented method of generating recommended cleaning and/or treatment characteristics for different parts of a mouth of a subject.

The computer-implemented method comprises: obtaining, for each of a plurality of first parts of the mouth of the subject, quality data indicating a quality of said first part of the mouth; and generating, for each of two or more second parts of the mouth of the subject, one or more recommended cleaning and/or treatment characteristics for said second part of the mouth by processing the quality data of any first parts of the mouth overlapping said second part of the mouth of the subject.

The present disclosure recognizes that previous approaches for recommending cleaning and/or treatment characteristics (such as brushing times or brushing pressure) fail to take into consideration the individual state of each tooth/quadrant/segment, or the distinction between healthy and diseased teeth. For instance, teeth with inflamed gums, pockets or caries are given the same recommended cleaning and/or treatment approach as healthy teeth. In other words, it is recognized that the non-personalization of current recommended cleaning and/or treatment characteristics could lead to certain areas of the subject's mouth being under cleaned/treatment (i.e. insufficiently cleaned/treated to achieve a desired oral standard) or be subject to potentially excess or unnecessary cleaning/treatment.

The proposed invention discloses a method to automatically set one or more recommended cleaning and/or treatment characteristics for different parts of a user's mouth based on a quality assessment of the user's mouth (and, in particular, parts/sections of the user's mouth). In this way, problematic areas or areas with high risk can get assigned different cleaning and/or treatment characteristics to healthy areas. This facilitates prioritization of the problematic areas, thereby improving oral hygiene, reducing the likelihood of oral problems and providing a better outcome for a patient.

The present disclosure uses image data of a subject to assess the quality of various first parts of the subject's mouth. This quality information is then used to determine or select at least one cleaning and/or treatment characteristic to be recommended for second parts of the subject's mouth. In particular, the quality information of any first parts of the subject's mouth overlapping (e.g. entirely and/or partially contained within) a particular second part are used to determine, assign or set a recommended characteristic for that second part.

The proposed approach generates information usable in carrying out a more successful technical task by a user, namely that of cleaning and/or treating teeth. There is therefore a technical purpose in generating data directed towards defining the parameters of a technical act to be carried out.

Cleaning is considered to relate to any activity for improving a hygiene of the oral cavity, e.g. brushing or flossing. Examples of suitable recommended cleaning and/or treatment characteristics include: a recommended time for cleaning and/or treatment (e.g. a recommended brushing time); a recommended pressure to be applied during a cleaning and/or treatment procedure (e.g. a pressure or (mechanical) motion to be applied by a toothbrush or flosser); a recommended direction for cleaning and/or treatment (e.g. a recommended direction in which to move a brush or flosser); a recommended pattern for performing cleaning and/or treatment and so on.

In some embodiments, for each of two or more second parts of the mouth of the subject, the step of generating one or more recommended cleaning and/or treatment characteristics comprises generating a recommended cleaning and/or treatment time. In particular, the recommended cleaning and/or treatment time may be a recommended cleaning time such as a recommended brushing time (for a toothbrush or any other oral care device including but not limited to flossing devices, combined brushing and flossing devices, whitening devices, full or partial cleaning mouthpieces, etc.).

The step of generating a recommended cleaning and/or treatment time for each second part of the mouth of the subject may comprise: obtaining a desired total cleaning and/or treatment time (e.g. for the entire mouth); distributing the desired total cleaning and/or treatment time between each second part of the mouth of the subject, based on the assessed quality of any first parts the mouth overlapping said second part of the mouth of the subject.

It is recognized that a subject usually has a desired total cleaning and/or treatment time (e.g. the recommended 2-minute brushing time), and that compliance may be increased if this time is not exceeded. In other words, excessive recommended times lead to reduced user compliance, and therefore reduced efficacy of cleaning and/or treatment over a period of time.

The step of generating a recommended cleaning and/or treatment time for each second part of the mouth of the subject may comprise determining a recommended cleaning and/or treatment time for each second part of the mouth of the subject, based on the assessed quality of any first parts of the mouth overlapping said second part of the mouth of the subject, independently of any other recommended cleaning and/or treatment times for any other second part of the mouth of the subject.

In some examples, each first part of the mouth of the subject is an area of the mouth of the subject that should contain only a single oral surface (e.g. including soft tissue) or only a single tooth.

An oral surface is any known surface of the oral cavity, and may be a tooth surface. In the context of the present application, each oral surface may have a smaller size than any tooth of the subject. A tooth surface is a part of a tooth (and could be alternatively labelled a "surface of a tooth"). Tooth surfaces have a distinct definition in the art to refer to specific areas of the tooth. Examples of tooth surfaces include: the distal surface; the facial surface; the labial surface; the buccal surface; the incisal surface; the lingual surface; the mesial surface; the occlusal surface; and the proximal surface. In some examples, an oral surface may be part of a gum or soft tissue surface, e.g. a part of the gum or soft tissue in the vicinity of a particular tooth.

In some examples, this facilitates individual assessment of each tooth of the subject (or position where a tooth should be present) to determine the health/disease state of the tooth. This approach provides a highly accurate mechanism for recommending one or more cleaning and/or treatment characteristic(s) for an area containing that tooth, e.g. as areas containing more than one problematic tooth (or a particularly problematic tooth) can be prioritized.

Optionally, each second part of the mouth of the subject represents: a different segment or quarter of the mouth of the subject; or a different tooth of the mouth of the subject. In particular example, the second part of the mouth may be larger (in size, volume, width, height, length) than the first part of the mouth. Of course, in other examples, the second part of the mouth is equal in size to a first part of the mouth.

The computer-implemented method may be adapted wherein: the quality data of each two or more first parts of the mouth of the subject comprises a ranking of said first parts of the mouth of the subject, wherein the ranking is based on a health or disease status of the said first parts of the mouth of the subject; and the step of generating the one or more recommended cleaning and/or treatment characteristics for each second part of the mouth of the subject is responsive to the rankings of any first parts of the mouth overlapping said second part of the mouth of the subject. In other words, the quality of a part of the mouth of the subject may be a relative quality, e.g. in the form of a ranking.

In some examples, the quality data of each two or more first parts of the mouth of the subject comprises an identification of any missing and/or additional teeth in each first part of the mouth of the subject; and the step of generating the one or more recommended cleaning and/or treatment characteristics for each second part of the mouth is responsive to the presence or absence of any missing and/or additional teeth in any first parts of the mouth of the subject overlapping said second part of the mouth of the subject.

There is a reduced need to clean/treat an area containing any missing teeth or clean longer areas with more teeth, as it would be more beneficial to perform cleaning and/or treatment on present teeth (e.g. to reduce the likelihood of further disease or the like). Contrary wise, there is an increased need to clean/treat an area containing any additional teeth (e.g. mesiodens or supernumerary teeth). Similarly, if a part of the mouth contains a tooth expected to be in another part of the mouth (e.g. an ectopic tooth or an impacted tooth), then this can be taken into account.

In some embodiments, the quality data of each two or more first parts of the mouth of the subject comprises an identification of any teeth with any restorations, in each first part of the mouth of the subject; and the step of generating one or more recommended cleaning and/or treatment characteristics for each second part of the mouth is responsive to the presence or absence of any (extra) teeth with restorations in any first parts of the mouth of the subject overlapping said second part of the mouth of the subject.

Dentally-restored teeth are any teeth that have undergone dental restoration, e.g. been subject to a filling, a crown, an implant, a replacement, a prosthetic tooth, a bridge or other dental prostheses. It is recognized that dentally-restored teeth would benefit from an increased brushing or flossing time. Accordingly, it would be advantageous to identify the presence of any dentally restored tooth in order to recommend an appropriate (e.g. increased) brushing time for any areas containing such dentally restored teeth.

Another example could be distinction of milk (primary) baby teeth from permanent teeth and adjustment of brushing time or flossing characteristics accordingly, while focus is given to permanent teeth. For example, kids do not need to necessarily floss baby teeth as there are always spaces between them (so self-cleansing to a certain degree). While for permanent teeth, the contact areas or points start to be shaped, so it is important to floss and more attention given to permanent teeth.

Thus, in some embodiments, the quality data of each two or more first parts of the mouth of the subject comprises an identification of any deciduous (i.e. baby, milk or primary) teeth, in each first part of the mouth of the subject; and the step of generating one or more recommended cleaning and/or treatment characteristics for each second part of the mouth is responsive to the presence or absence of any deciduous teeth in any first parts of the mouth of the subject overlapping said second part of the mouth of the subject. It is recognized that parts of the mouth containing deciduous teeth require less attention during cleaning and/or treatment than parts containing permanent teeth.

In some examples, the quality data of two or more first parts of the mouth of the subject comprises a classification of a risk, health status and/or disease status of each first part of the mouth of the subject; and the step of generating one or more recommended cleaning and/or treatment characteristics for each second part of the mouth is responsive to any classifications of any first parts of the mouth of the subject overlapping said second part of the mouth of the subject.

The size of each first part of the mouth may be smaller than or equal to the size of any of the second parts of the mouth. In some examples, each first part of the mouth may overlap a single second part of the mouth, e.g. be entirely contained within a particular second part of the mouth.

The computer-implemented method may further comprise generating a first subject-perceptible output representing the one or more recommended cleaning and/or treatment characteristics for each second part of the mouth of the subject.

In this way, a subject may be given feedback about their recommended brushing times based on additional quality data indicative for segments or part requiring extra notion. This provides the subject with information to aid them in performing a technical task of brushing their teeth, with an outcome of improved oral hygiene.

There is also proposed a computer-implemented method of monitoring a cleaning and/or treatment of a mouth of a subject, the computer-implemented method comprising: obtaining the one or more recommended cleaning and/or treatment characteristics for different parts of the mouth of the subject generated by any previously described method, wherein the one or more recommended cleaning and/or treatment characteristics includes a cleaning and/or treatment time; monitoring a cleaning and/or treatment of the second parts of the mouth of the subject; and for each second part of the mouth of the subject, controlling a second subject-perceptible output based on whether or not a cleaning and/or treatment of said second part of the mouth of the subject exceeds a recommended cleaning and/or treatment time.

In this way, a subject is provided with active feedback about their cleaning and/or treatment.

There is also proposed a computer program product comprising computer program code means which, when executed on a computing device having a processing system, cause the processing system to perform all the steps of any herein described method. The computer program product may be formed from a (non-transitory) computer-readable medium.

There is also proposed a processing arrangement configured to generate recommended brushing, cleaning and/or treatment times for different parts of a mouth of a subject, the processing arrangement being configured to: obtain image data representing a mouth of a subject; process the image data to determine a quality of two or more first parts of the mouth of the subject; and generate, for each of two or more second parts of the mouth of the subject, a recommended cleaning characteristics (e.g. time) for said second part of the mouth based on the assessed quality of any first parts of the mouth overlapping said second part of the mouth of the subject.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 is a flowchart illustrating a method according to an embodiment;
Figure 2 is a flowchart illustrating a process usable in an embodiment;
Figure 3 is a flowchart illustrating a method according to an embodiment;
Figure 4 illustrates one example of a subject-perceptible output;
Figure 5 illustrates a method for use in an embodiment;
Figure 6 illustrates a workflow that makes use of a method according to an embodiment;
Figure 7 illustrates a processing arrangement according to an embodiment; and
Figure 8 illustrates a processing system according to an embodiment.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an approach for generating recommended cleaning and/or treatment characteristics for different parts of a subject's mouth. Quality data about two or more first parts of the mouth is obtained. At least one cleaning and/or treatment characteristic for each of two or more second parts of the mouth are generated by (for each second part) processing the quality data of any overlapping first parts of the mouth.

Embodiments of the invention are based on the realization that part (e.g. region or area) specific recommendations about cleaning and/or treatment would assist a subject (or other person) in achieving improved cleaning and/or treatment of the subject's mouth. In particular, more granular guidance is provided to effectively identify areas of concern and how to adapt a cleaning and/or treatment procedure to respond to such areas of concern.

Embodiments may be employed, for example, to guide tooth brushing for a subject in a home environment or to guide cleaning and/or treatment in a clinical (dental) environment. Embodiments could be integrated into smart oral care applications, e.g. connected oral care devices.

In the context of the present disclosure, the terms "subject", "user" and "patient" are considered to be interchangeable.

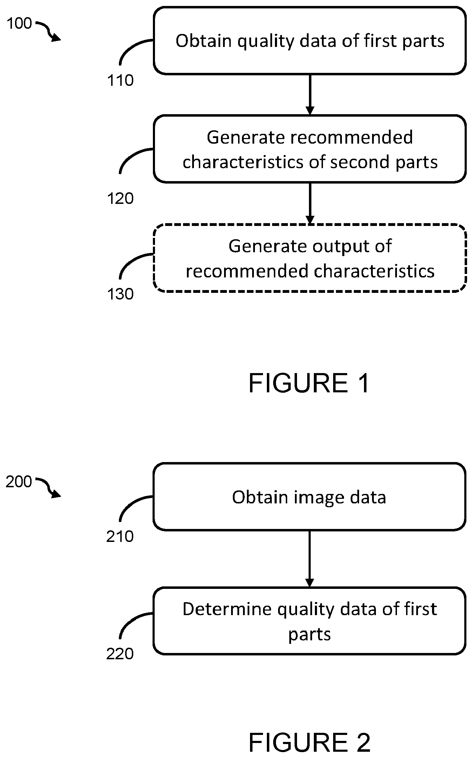

Figure 1 is a flowchart illustrating a (computer-implemented) method 100 of a generic embodiment.

The method 100 comprises a step 110 of obtaining, for each of a plurality of first parts of the mouth of the subject, quality data indicating a quality of said first part of the mouth.

The mouth of the subject is thereby conceptually divided into a plurality of first parts. Step 110 obtains quality data for each first part of the mouth. Quality data may contain any binary, numeric or categorical measure of a quality (e.g. condition, state, presence etc.) of the first part of the mouth. The quality data thereby comprises any subject-specific data that defines a measure or indicator of quality of a corresponding first part of the user's mouth, and may comprise one or more data points that represent a quality of a first part of the mouth of the subject.

The quality data may be obtained by processing some (raw) data about the user's mouth, e.g. by processing obtained image data. Alternatively, the quality data may be obtained from a memory or storage system, e.g. from the subject's medical records. For instance, quality data may be derived from a dental record of the subject (e.g. indicating a condition and/or presence/absence of different first parts of the mouth, e.g. different teeth). As yet another example, the quality data may be obtained from a user input, e.g. a user inputting information about the health of their teeth or gums - e.g. indicating a condition and/or presence/absence of different parts of the mouth).

A first part of the mouth may comprise, for instance, an oral surface site (e.g. a particular part or surface of a tooth or oral cavity) or an area of the mouth that should contain a single (real or prosthetic) tooth, i.e. an area of the mouth containing a single tooth socket and (if present) anything mounted in the tooth socket, such as a tooth or dental implant. Of course, it will be appreciated that the first part of the mouth may be larger, e.g. a segment or quadrant of the mouth.

An oral surface is any known surface of the oral cavity, and may be a tooth surface. A tooth surface is a part of a tooth (and could be alternatively labelled a "surface of a tooth"). Tooth surfaces have a distinct definition in the art to refer to specific areas of the tooth. Examples of tooth surfaces include: the distal surface; the facial surface; the labial surface; the buccal surface; the incisal surface; the lingual surface; the mesial surface; the occlusal surface; and the proximal surface. In some examples, an oral surface may be part of a gum or soft tissue surface, e.g. a part of the gum or soft tissue in the vicinity of a particular tooth.

Various examples for content of quality data are hereafter described. Quality data may contain a single one of such examples, a combination of such examples or a single value/indicator derived from combining or further processing such examples.

By way of example, quality data may comprise a classification of the first part of the mouth (e.g. as output by a machine-learning method). The classification may, for instance, be a classification of a risk, health status and/or disease status of the first part of the mouth.

As another example, quality data may comprise a ranking of the first part of the mouth (compared to other first parts of the mouth). The ranking may, for instance, be responsive to a risk, health status and/or disease status of the first part of the mouth. By way of example, a ranking may represent a relative rank of amount of plaque, disease (e.g. periodontal disease) decay, cavities (caries) or other suitable measures of health/disease status.

As yet another example, quality data may comprise an identification of any missing (real and/or prosthetic) or extra teeth in each first part of the mouth of the subject. This may, for instance, be a numeric measure of missing teeth or a binary measure indicating whether or not any teeth are missing.

As yet another example, quality data may comprise an identification of any teeth with any (dental) restorations. This may, for instance, be a numeric measure of teeth with restoration, a numeric measure of a number of restorations, a binary measure indicating whether or not there are any teeth with dental restorations and/or a binary measure indicating whether or not there are any dental restorations.

As yet another example, quality data may comprise an identification of any additional teeth, e.g. any mesiodens, supernumerary teeth, ectopic teeth or impacted teeth. This may, for instance, be a numeric measure of additional teeth and/or a binary measure indicating whether or not there are any additional teeth.

As yet another example, quality data may comprise an identification of any deciduous teeth, i.e. any baby, milk or primary teeth. This may, for instance, be a numeric measure of deciduous teeth and/or a binary measure indicating whether or not there are any deciduous teeth.

Other suitable examples of data suitable for forming (part of the) quality data will be apparent to the skilled person, e.g. a measure of decay in the first part of the mouth, a measure of plaque in the first part of the mouth, a measure of (average) enamel thickness in the first part of the mouth and so on.

The method 100 further comprises a step 120 generating, for each of two or more second parts of the mouth of the subject, one or more recommended cleaning and/or treatment characteristics for said second part of the mouth. Each recommended cleaning and/or treatment characteristic is calculated by processing the quality data of any first parts of the mouth overlapping said second part of the mouth of the subject.

Thus, step 120 comprises generating recommended cleaning and/or treatment characteristics (for a second part of a subject's mouth) based on the quality data of first parts of the mouth overlapping, e.g. contained with, the second part of the mouth of the subject. Examples of a recommended cleaning and/or treatment characteristic include: a recommended time for cleaning and/or treatment (e.g. a recommended brushing time); a recommended pressure to be applied during a cleaning and/or treatment procedure (e.g. a pressure or (mechanical) motion to be applied by a toothbrush or flosser); a recommended direction for cleaning and/or treatment (e.g. a recommended direction in which to move a brush or flosser); a recommended pattern for performing cleaning and/or treatment and so on.

Thus, step 120 generates bespoke guidance for different parts of the mouth of the subject (i.e. provides non-generic guidance for brushing the mouth of the subject). Thus, guidance is specifically adapted for different areas of the subject's mouth. This provides useful clinical information for improving the performance of a cleaning/treatment of the subject's mouth. In particular, the guidance provides information on areas that require closer or more careful attention during a cleaning and/or treatment procedure, to credibly aid a subject in the performance of a technical task with an improved outcome.

Each second part of the mouth of the subject may be equal in size or larger than any given first part of the mouth of the subject. Thus, the size of each first part of the mouth is smaller than or equal to the size of any of the second parts of the mouth. In some examples, each first part of the mouth of the subject acts as a second part of the mouth of the subject.

Preferably, each first part of the mouth is entirely contained within a second part of the mouth (i.e. overlaps only a single second part of the mouth).

The second part of the mouth may, for instance, be a quadrant (i.e. a quarter) or a segment/fraction (e.g. a sixth, an eighth, a tenth, a twelfth, a sixteenth and so on) of the mouth. In other examples, the second part of the mouth may be an area of the mouth of the subject that should contain only a single oral surface or only a single tooth. In particular examples, a first part of the mouth may also act as a second part of the mouth.

In one example, step 120 comprises determining, for each second part of the mouth, a recommended pressure to be applied when brushing the second part of the mouth based on the quality data of any overlapping first parts. For instance, a first (lower) pressure may be recommended if the quality data identifies that an average enamel thickness or hardness in the second part of the mouth is below some predetermined threshold and a second (higher) pressure may be recommended if the quality data identifies that an average enamel thickness in the second part of the mouth is above some predetermined threshold. This approach takes account of likely sensitivity of the second parts of the mouth to improve a user experience.

In another example, step 120 comprises determining, for each second part of the mouth, a recommended fluid pressure to be applied by an oral irrigator during an oral irrigation process. For instance, a first (lower) pressure may be recommended if the quality data of overlapping first parts indicates that the second part contains below a predetermined threshold of plaque and a second (higher) pressure may be recommended if the quality data of overlapping first parts indicates that the second part contains above a predetermined threshold of plaque. This approach can allow for more efficient use of oral irrigation resource (e.g. water, power and so on) whilst still allowing higher pressure to be applied when there is a larger amount of plaque.

Other approaches for generating recommended cleaning and/or treatment characteristics are envisaged, a number of which are described later in this document.

The method 100 may further comprise a step 130 of generating a first subject-perceptible output representing the one or more recommended cleaning and/or treatment characteristics for each second part of the mouth of the subject.

Step 130 may, for instance, comprise controlling a user interface to provide a user-perceptible output (e.g. an audio, visual, olfactoric and/or haptic output) representing the one or more recommended cleaning and/or treatment characteristics. The precise operation performed by step 130 may depend upon the nature of the user interface, as would be readily apparent to the skilled person.

Providing the proposed guidance (recommended cleaning and/or treatment characteristics) to the subject via the user interface provides a guided human-machine interaction process to assist the subject in the performance of a technical task of cleaning and/or treating the teeth. By taking account of the provided guidance, the user is able to perform an improved and/or more subject-specific cleaning and/or treatment approach.

In some examples, the method 100 may comprise a step (not shown) of storing the recommended cleaning and/or treatment characteristics in a memory or storage device.

Figure 2 is a flowchart illustrating a (computer-implemented) method 200 of obtaining quality data of each of a plurality of first parts of the subject's mouth. The method 200 is only one example of such a method for use in embodiments, e.g. for use in step 110 described with reference to Figure 1.

The method 200 comprises a step 210 of obtaining image data representing a mouth or oral cavity of a subject.

The image data should contain sufficient data representing the mouth of the subject to facilitate automated assessment of the quality (e.g. condition, state, presence and so on) of one or more first parts of the mouth of the subject. Suitable image data may include x-ray data of the subject's mouth or intraoral image data of the subject's mouth, i.e. data obtained from imaging the interior of the subject's mouth. The intraoral image data may be any suitable image captured, e.g. using a smartphone camera, a compact camera, a dedicated intraoral imaging device and so on.

The image data may be obtained directly from a medical imaging device (e.g. an X-ray scanner, an intraoral scanner, CT-scanner, Ultrasound imaging device or MRI equipment), or from a memory or storage device (e.g. that stores data generated by such a medical imaging device). The image data may contain, for instance, X-ray data, CT data, ultrasound data, MRI data or intraoral scanner data (such as optical coherence tomography data or visible light image data).

The method 200 also comprises a step 220 of processing the image data to determine quality data for each of two or more first parts of the mouth of the subject. Step 220 may be performed using an automated process, e.g. to assess and identify one or more indicators of quality for each first part of the mouth of the subject. Examples of suitable quality data have been previously described. The automated process may, for instance, comprise performing a segmentation algorithm on the image data to identify characteristics of the first parts for assessing a quality of the first parts.

In one example, step 220 comprises identifying any missing (real or prosthetic) or extra teeth in each first part of the mouth of the subject by processing the image data, e.g. using a segmentation algorithm. Thus, the quality data may comprise an identification of any missing (real and/or prosthetic) teeth in each first part of the mouth of the subject - such as numeric measure (i.e. a number) of missing or absent teeth, or a binary indicator of whether or not there are any missing teeth. Identification of any missing teeth could be performed by using automated segmentation technique (e.g. an appropriately trained machine-learning method or a model fitting technique) to identify the presence and position of any teeth in the mouth, and processing the segmentation result to determine, for each first part, an indicator of whether there are any missing teeth in the first part.

In an example, step 220 comprises identifying any teeth with restorations, in each first part of the mouth of the subject by processing the image data. Thus, the quality data may comprise an identification of any dentally restored teeth (teeth with restoration) in each first part of the mouth of the subject - such as numeric measure (i.e. a number) of teeth with restorations, or a binary indicator of whether or not there are any teeth with restorations. Dentally-restored teeth are any teeth that have undergone dental restoration, e.g. been subject to a filling, a crown, an implant, a replacement, a prosthetic tooth, a bridge or other dental prostheses.

In an example, step 220 comprises identifying any additional teeth, in each first part of the mouth of the subject by processing the image data. Thus, the quality data may comprise an identification of any additional teeth in each first part of the mouth of the subject - such as numeric measure (i.e. a number) of additional teeth, or a binary indicator of whether or not there are any additional teeth.

In an example, step 220 comprises identifying any deciduous teeth, in each first part of the mouth of the subject by processing the image data. Thus, the quality data may comprise an identification of any deciduous teeth in each first part of the mouth of the subject - such as numeric measure (i.e. a number) of deciduous teeth, or a binary indicator of whether or not there are any deciduous teeth.

Where step 220 comprises identifying any missing/additional/deciduous teeth and/or any teeth with restorations, the image data may be segmented using a segmentation algorithm (to identify areas representing each first part of the mouth) before each area segmented area using a further segmentation technique (to identify the above-mentioned teeth).

In some embodiments, step 220 comprises classifying a risk, health status and/or disease status of each first part of the mouth of the subject. By way of example, a classified risk may, for instance, comprise a classified risk score (e.g. a categorical measure or numeric measure of predicted health risk). A classified risk score could, for instance, be a score on a scale of 0-10, 1-10, 0-100, 1-100, 0-1 and so on, or a categorical score (e.g. "High", "Low", "Medium").

Thus, step 220 may comprise processing the image data using a classification technique (such as a machine-learning method, e.g. a neural network) to generation a classification for each first part of the mouth of the subject. This process may comprise segmenting the image data to identify areas representing each first part of the mouth (e.g. using a segmentation algorithm), before processing each segmented area using a classification technique (which may be generic or specific to said segmented area) to generate the classification for each first part of the mouth.

Where segmentation is performed, any suitable segmentation technique may be used to, e.g. a Hankel transformation algorithm, a deformable contour segmentation algorithm and/or a machine-learning method. As such algorithms are well-known per se, the segmentation technique will not be explained in further detail for the sake of brevity only.

In yet another working example, step 220 may comprise identifying one or more at-risk areas in the first part, e.g. areas that would require additional cleaning/treatment, such as areas of dental restoration, dental implants, dental crowns, fillings and so on. An automated technique for performing such a process could be used, e.g. a machine-learning method.

In another working example, where each first part of the mouth represents a tooth of the subject, a quality of the first part of the mouth may be performed by quantifying an amount of plaque on each tooth, e.g. using the approach suggested by Carter, Kevin, Gabriel Landini, and A. Damien Walmsley. "Automated quantification of dental plaque accumulation using digital imaging." Journal of dentistry 32.8 (2004): 623-628. Of course, where a first part of the mouth comprises represents more than one tooth, a measure of plaque for each tooth in the first part of the mouth may be calculated and then combined (e.g. summed or averaged) to determine a measure of plaque in the first part of the mouth.

In yet another working example, where each first part of the mouth represents a tooth of the subject, a quality of the first part of the mouth may be performed by detecting and scoring caries, e.g. using the approach suggested by Yin, Myat Su, et al. "Automated outcome scoring in a virtual reality simulator for endodontic surgery." Computer methods and programs in biomedicine 153 (2018): 53-59. Of course, where a first part of the mouth comprises represents more than one tooth, a score for any caries in the first part of the mouth may be calculated and then combined (e.g. averaged or, more preferably, summed).

In another working example, a quality of a first part of the mouth may be performed by detecting an (average) enamel thickness, e.g. using the approach suggested by Koprowski, Robert, et al. "Automatic method of analysis of OCT images in the assessment of the tooth enamel surface after orthodontic treatment with fixed braces." Biomedical engineering online 13.1 (2014): 1-19. Of course, where a first part of the mouth comprises represents more than one tooth, an enamel thickness for each tooth in the first part of the mouth may be calculated and then combined (e.g. averaged).

A combination of these approaches may be used. For instance, quality data of a first part of the mouth of the subject may comprise any combination of the previously mentioned examples for content of quality data (e.g. an indicator of any absent teeth and a measure of plaque). In some instances, quality data may comprise a single value calculated by processing a variety of quality data inputs values (e.g. by combining, e.g. weighting, any previously described examples for content of quality data).

Performance of the method 200 is not essential to achieve embodiments of the invention, but rather serves to demonstrate how quality data could be automatically generated from image data of the subject. Quality data may be obtained using other approaches, e.g. responsive to a user input (providing information about the quality of their teeth) and/or retrieving information from a memory or storage (e.g. storing dental records of the patient, containing quality information of first parts of the subject's mouth). The quality data used in embodiments may contain any of the possible parts of quality data described above.

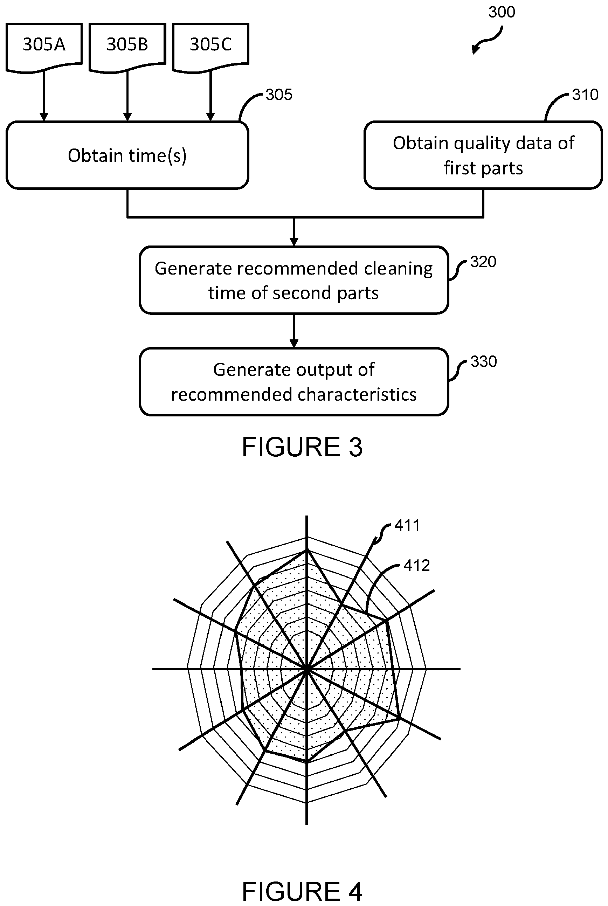

Figure 3 illustrates a method 300 according to an example of the invention, which provides a specific working example of an embodiment of the invention.

The method 300 generates a recommended brushing time for each of a plurality of second parts of the mouth of a subject. The described approach could be readily adapted for generating another form of recommended cleaning and/or treatment characteristic, and in particular, another form of recommended cleaning and/or treatment time. Thus, reference to a "brushing time" could be replaced by a "cleaning and/or treatment time", or by "oral hygiene time" where appropriate.

The method 300 comprises a step 305 of obtaining a desired total brushing time 305A. The desired total brushing time may be a predetermined or preset time, e.g. a globally recommended time such as 2 minutes, or a time provided responsive to a user input (e.g. a user-desired brushing time).

The method 300 comprises a step 310 of obtaining, for each of a plurality of first parts of the mouth of the subject, quality data indicating a quality of said first part of the mouth.

The method 300 comprises a step 320 of processing the obtained quality data to generate a recommended brushing time for each second part of the mouth. Step 320 comprises distributing the total brushing 305A between the second parts of the mouth based on the quality data of the first parts of the mouth overlapping the second part of the mouth.

Step 320 could be performed, for instance, by distributing a desired brushing time between the different second parts of the mouth based on a risk score of each second part of the mouth, such as a categorical risk score. Here a risk score may be a risk of further disease or breakages in the second parts of the mouth. A risk score for each second part of the mouth could be derived from the quality data of any first parts of the mouth overlapping said second part of the mouth. In some examples, further characteristics of the subject (who has the mouth) may be used in determining the risk score, such as an age, gender, presence or absence of diabetes, dental history, medical history and/or conditions and so on.

For instance, a categorical risk score for each second part of the mouth could be derived from the risk scores of the overlapping first parts of the mouth, e.g. by (for each second part) taking a modal categorical risk score of the risk scores of first parts contained in the said second part or by further processing the categorical risk scores of the first parts contained in the second part according to some algorithm, such as an if-this, then-that algorithm (e.g. if a second part contains more than X many first parts having a "High" risk score, then the second part is assigned a "High" risk score - where X is some predetermined value).

As another example, a categorical risk score for each second part of the mouth could be derived from an indicator of any missing and/or dentally restored and/or additional and/or deciduous teeth in the mouth. For instance, the presence of dentally restored teeth may increase a risk whereas the presence of missing teeth may decrease a risk. As another example, the presence of deciduous teeth may decrease a risk. In one example, if a number of dentally restored teeth in the second part of the mouth exceeds some predetermined value, the categorical risk score is determined to be "High".

This categorical risk score can then be used to determine a weighting factor for each second part of the mouth. For instance, a "High" risk score can be assigned weighting factor of 3, a "Medium" risk score can be assigned a weighting factor of 2 and a "Low" risk score can be assigned a weighting factor of 1. The weighting factor thereby effectively represents a numeric indicator of the categorical risk score.

The weighting factors are then usable to distribute a total brushing time across the different second parts of the mouth. For instance, a total brushing time (e.g. a predetermined total brushing time or, if obtained, the desired brushing time 305A) may be divided by the summed total of the weighting factors to determine a base brushing time. The base brushing time may then, for each second part, be multiplied by the weighting factor of the second part to determined a recommended brushing time for the second part.

Table 1 illustrates an example of this approach, in which the second parts of the mouth are segments representing a different sixth of the mouth and a total brushing time is 120s (2 minutes).

**TABLE 1**

| Segment | 1 | 2 | 3 | 4 | 5 | 6 |
|---|---|---|---|---|---|---|
| Risk | High | Low | High | Low | Medium | Medium |
| Weighting | 3 | 1 | 3 | 1 | 2 | 2 |
| Brushing Time (s) | 30 | 10 | 30 | 10 | 20 | 20 |

Instead of using (categorical) risk score, other suitable scores or measures (e.g. measures contained in the quality data of first parts of the mouth overlapping the respective second part) could be used to select a weighting factor for the second part of the mouths.

As a simple example, consider a scenario in which quality data of first parts overlapping a particular second part identifies a number of teeth with any restorations. A first (lower) weighting factor, e.g. 1, may be selecting if the second part contains no teeth with any restorations and a second (higher) weighting factor, e.g. 2, may be selected if the second part contains at least one teeth with any restorations. In a continuation of this example, a third (yet higher) weighting factor, e.g. 3, may be selected if the second parts contains more than one tooth with any restorations.

As another example, the quality data of the first parts may be used to determine a relative ranking of each second part with respect to one another. A ranking of each second part may be used to determine its weighting factor. For instance, a highly ranked second part (i.e. highest quality) may have a lower weighting factor than a lowly ranked second part. The weighting factors may be inverse to the ranking (where 1 represents high quality and N represents low quality, N being greater than 1).

A combination of different elements of quality data of overlapping parts may be used to determine a weighting factor for a second part of the mouth.

In another example of step 320, a recommended brushing time may be individually calculated for each second part of the mouth.

Each recommended brushing time may then be scaled by a ratio between the total recommended brushing time (being the sum of the individually calculated brushing times) and the desired total brushing time, so that the sum of the scaled recommended brushing times is equal to the desired total brushing time.

In one example, an individually calculated recommended brushing time for second part of the mouth is determined based on a number of missing and/or deciduous teeth and/or a number of teeth with restorations in the second part of the mouth (e.g. as identified in the quality data of any overlapping first parts).

For instance, a desired brushing time may be divided by the total number of (non-missing) teeth in the mouth of the subject to produce a per-tooth brushing time. A baseline recommended brushing time for each second part may be calculated by multiplying the number of teeth in the second part by the per-tooth brushing time. An individually calculated brushing time for the second part can be calculated by adding a predetermined additional time to the baseline recommended brushing time based on a number of teeth with restorations in the second part, e.g. adding an extra 5 seconds for each tooth with one or more restorations in the second part. Optionally, if used, each deciduous tooth in the second part of the mouth may subtract a second predetermined additional time, e.g. subtracting 3 seconds for each deciduous tooth in the second part.

In another example of step 320, a recommended brushing time for each second part could be determined based on recommended brushing times for the first parts of the mouth. Thus, a recommended brushing time may be calculated for each first part, which can then be processed to determine the recommended brushing time for each second part.

For instance, a desired brushing time for each first part may be divided by the total number of (non-missing) teeth in the mouth of the subject to produce a per-tooth brushing time. In this scenario, each first part of the mouth is effectively a part that should contain a single tooth, so that a recommend brushing time is generated for each tooth (i.e. if the first part contains a tooth, it has a recommended brushing time of the per-tooth brushing time, and if it does not contain a tooth, it has a recommended brushing time of 0).

A recommended brushing time for each second part can then be derived by summing the recommended brushing times of any first part overlapping or contained within said second part.

In the method 300, a predetermined brushing time is distributed between different second parts of the mouth responsive to quality data of first parts overlapping the second parts.

In some examples, there is a minimum brushing time 305B for each second part of the mouth. In this scenario, if a distributed brushing time for a second part falls below the minimum brushing time, then step 320 may be configured such that the recommended brushing time for that second part is the minimum brushing time. If this occurs then the total recommended brushing time (i.e. the combination of all recommended brushing times for the second parts) will exceed the desired total brushing time. The minimum brushing time may be predetermined and/or obtained in step 305.

In some examples, there may be a maximum total brushing time 305C. This may be obtained in step 305 or be predetermined. In this example, if the total recommended brushing time exceeds the maximum total brushing time, then the recommended brushing times for the second parts may be reduced (e.g. scaled down) until the total recommended brushing time falls below the maximum total brushing time. In some examples, where there is a minimum brushing time 305B for each second part, then a recommended brushing time for any given second part may not fall below this minimum brushing time, so that only those recommended times that exceed the minimum brushing time are scaled down until the total recommended brushing time falls below the maximum total brushing time.

Method 300 may further comprise a step 330 of generating a first subject-perceptible output representing the one or more recommended cleaning and/or treatment characteristics for each second part of the mouth of the subject. This may be performed using the same approach 130 as previously described.

Figure 4 illustrates one example of a subject-perceptible output 400 representing the recommended brushing times. Here, the subject-perceptible output is a visual output, representing a recommended time 412 for brushing each of twelve segments 411 of the mouth. Concentric polygons illustrates different recommended brushing times, in which an outermost polygon indicates a greater recommended brushing time than an innermost polygon. A position of a brushing time indicator 412 on a line 411 representing a particular segment illustrates a recommended brushing time for that segment.

The visual output 400 may be provided at a user interface, e.g. an output display. The previously described method 300 describes an approach for distributing a desired total brushing time between different second parts of the mouth. However, in other examples, there is no need to perform a distribution, and each second part of the mouth may be assigned a specific (i.e. individual) recommended brushing time based on quality data of overlapping first parts. Thus, the total brushing time may be variable, rather than fixed.

By way of example, each second part of the mouth may be assigned a predetermined baseline brushing time (e.g. a minimum of 20 seconds for a sixth of the mouth or 30 seconds for a quadrant). Quality data of overlapping first parts may determine how much additional time is added, or whether any time is subtracted, from this predetermined baseline brushing time.

For instance, each tooth with restorations in the second part (as identified by appropriate quality data of overlapping first parts) may add first additional time (e.g. 5 seconds or 10 seconds) to the time for the second part. As another example, the presence of caries or dental cavities in the second part (as identified by appropriate quality data of overlapping first parts) may add second additional time (e.g. 5 seconds or 10 seconds) to the time for the second part. As yet another example, a quantity of plaque (as identified by appropriate quality data of overlapping first parts) may defined a third additional time to be added to the time for the second part. The magnitude of the third additional time may be dependent upon the quantity of plaque, so that the greater the quantity of plaque, the greater the third additional time.

There is no need to incorporate all of these exemplary additional times, and other suitable examples for determining additional time(s) will be readily apparent to the person skilled in the art.

As one example, each missing tooth in a second part of the mouth may subtract a predetermined time period from the predetermined baseline brushing time, e.g. subtract 5 seconds for a sixth of the mouth or 8 seconds for a quadrant. In one example, each deciduous tooth in a second part of the mouth may subtract another predetermined time period from the predetermined baseline brushing time, e.g. subtract 3 seconds for a sixth of the mouth or 5 seconds for a quadrant. These predetermined time periods are merely examples and may be modified according to implementation details.

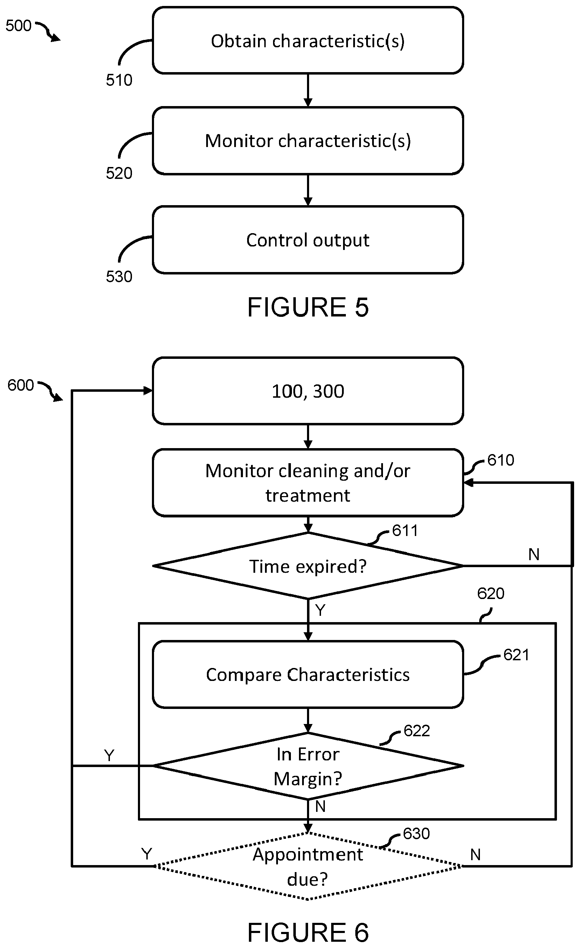

Figure 5 illustrates a method 500 for monitoring a cleaning and/or treatment of a mouth of a subject.

The method 500 comprises a step 510 obtaining the one or more recommended cleaning and/or treatment characteristics for different parts of the mouth of the subject generated by a previously described method, e.g. method 100 or method 300. This step may be performed by executing method 100 or method 300.

The method 500 further comprises a step 520 of monitoring a cleaning and/or treatment of the second parts of the mouth of the subject. For instance, where the recommended cleaning and/or treatment characteristic comprises a recommended cleaning and/or treatment time, this may comprise monitoring for how long each second part of the mouth undergoes cleaning and/or treatment. Where the cleaning and/or treatment characteristics comprises a recommended cleaning pressure (e.g. brush pressure), this may comprise monitoring a pressure applied to each second part of the mouth.

Approaches for monitoring cleaning and/or treatment characteristics are being met may make use of position and/or location monitoring (to identify a second part of the mouth undergoing cleaning/treatment). Such position and/or location monitoring approaches are known in the art and may make use, for example, of motion, movement and/or position data of a cleaning/treatment device such as a toothbrush. US 2014/246049 A1, EP 3,692,858 A1 and US 2021/010800 A1 describe various approaches for assessing the position of a device with respect to the body.

Approaches for assessing a characteristic of a cleaning and/or treatment will be readily apparent to the skilled person. For instance, where the characteristic is a time, a stopwatch module may be used. Where the characteristic is a pressure, a pressure-sensing device may be used. Where the characteristic is a direction of cleaning/treatment, position/movement data (e.g. from an accelerometer) may be monitored

The method 500 further comprises a step 530 of, for each second part of the mouth of the subject, controlling a second subject-perceptible output responsive to whether or not a cleaning and/or treatment of said second part of the mouth of the subject meets the recommended cleaning and/or treatment characteristics, e.g. whether or not a (monitored/actual) cleaning and/or treatment time exceeds a recommended cleaning and/or treatment time.

In this way, the subject is provided with feedback on whether they are adhering to the recommended cleaning and/or treatment characteristics.

Fig. 6 illustrates a larger workflow 600 that incorporates embodiments of the previously described method.

The workflow performs a previously described method 100, 300 to calculate recommended cleaning and/or treatment brushing times characteristics for different (second) parts of a mouth of a subject.

The workflow then moves to a process 610 of monitoring a cleaning and/or treatment process of the subject. In particular, the process monitors one or more characteristics of the cleaning and/or treatment for each second part of the mouth, and in particular the characteristics for which recommended characteristics were provided by method 100, 300. Thus, throughout process 610, the subject performs a cleaning and/or treatment procedure, e.g. brushes their teeth.

Process 610 is repeatedly performed for different cleaning and/or treatment sessions over the course of a predetermined time period, e.g. over the course of 2-3 months (e.g. typical recommended brush head replacement cycle). Expiration of this predetermined time period is checked (?) in step 611. If the predetermined time period has expired, the workflow moves to process 620, otherwise, the workflow reverts back to process 610 (in preparation for a next cleaning and/or treatment session).

The predetermined time period may be chosen based on the expected time it takes for clinical improvements or changes to the mouth when following the recommended one or more characteristics brushing times. This predetermined time period may be derived from a larger pool of data, e.g. of a user population. This large pool of data may be obtained over a period of time by gathering (for a substantial user population) the cleaning and/or treatment characteristics to be recommended in method 100, 300.

Process 620 comprises a step 621 of comparing the monitored actual characteristics to the recommended characteristics. If the monitored characteristic(s) for each second part of the mouth is outside of a predetermined error margin of the recommended characteristic, as detected in in a step 622, the method may revert back to method 100, 300 (e.g. new quality data for each first part of the mouth may be reobtained e.g. by rescanning the oral cavity or re-accessing medical records), and new recommended one or more characteristics may be generated).

By way of example, if a monitored and recommended characteristics is a brushing time, process 620 may comprise determining in a step 622 whether the monitored brushing times (for each second part) are within ±5% (or some other predetermined percentage, e.g. 10%) of the recommended brushing times.

If the monitored characteristic(s) fall within the predetermined error margin(s), the method may revert back to process 610 for another predetermined time period.

In some examples, the workflow 600 may also include a step 630 of determining whether a dental appointment is expected or advised (e.g. based on a standard recommendation for dental appointments, e.g. six months). A visit to a dental practitioner may be recommended if a time since a last dental appointment exceeds some predetermined time period. This step 630 is illustrated as taking place after step 622 (if the monitored characteristics are not within the error margin), but may be equally positioned at any other point in the workflow 600, e.g. between step 610 and 611 or between step 611 and process 620. If during a visit in the dental office new trouble spots (e.g. parts with excessive tartar/calculus formation, receeding gums etc) are found, these can be used to generate new quality data according to which the recommended brushing times (cleaning characteristics) are dynamically readjusted.

One possible workflow that makes use of a method is hereafter described. In this workflow, the method produces a recommended brushing time for each second part of the mouth. For this workflow, the recommended brushing times are displayed with respect to an oral image representing the mouth of the subject.

The workflow comprises a first step (or initiation step) in which non-optimized brushing times (e.g. globally recommended brushing times) are mapped onto the oral scan image from which quality data is generated (e.g. trouble spots identified). Generation of quality data may follow any previously described approach, and may include the annotation of low, medium, high risk teeth.

The workflow then moves to a second step (or dynamic adjustment step), in which the recommended brushing time for each second part are generated. Thus, the workflow determines the adjusted recommended brushing times for annotated (incl. critical high risk) areas. The adjusted recommended brushing time per second part can be based on a fixed total brushing time or for a variable total brushing time (i.e., by increasing the non-optimized brushing time in critical areas).

The workflow then moves to a third step, in which the user brushes with the recommended adjusted brushing times during a well-defined period until the end of this time period is reached. If the end of the time period is reached, the actual brushing time per segment is compared with the recommended brushing times.

The actual brushing data is then analyzed in a fourth step. If the actual brushing times are within ± 5% (or other predetermined error margin, e.g. ± 10%) of the proposed adjusted times, the system checks (in a fifth step) whether the next regular dental practitioner check (usually every 6 or 12 months) is due. If this is the case, an appointment will be automatically scheduled (in a sixth step) during which a new oral scan is made and potentially new trouble spots can be identified (i.e. in a seventh step). If new trouble spots are identified, a new annotation is made. If not, the system uses the adjusted brushing times as previously defined.

If the actual brushing times are not within ± 5 % (or other predetermined error margin0 of the proposed adjusted times, a re-scan appointment will be automatically scheduled (i.e. the sixth step will be performed) during which a new oral scan is made and potentially new trouble spots can be identified and the existing ones re-evaluated (seventh step). If new trouble spots are identified, a new annotation is made. If not, the system uses the adjusted brushing times as previously defined.

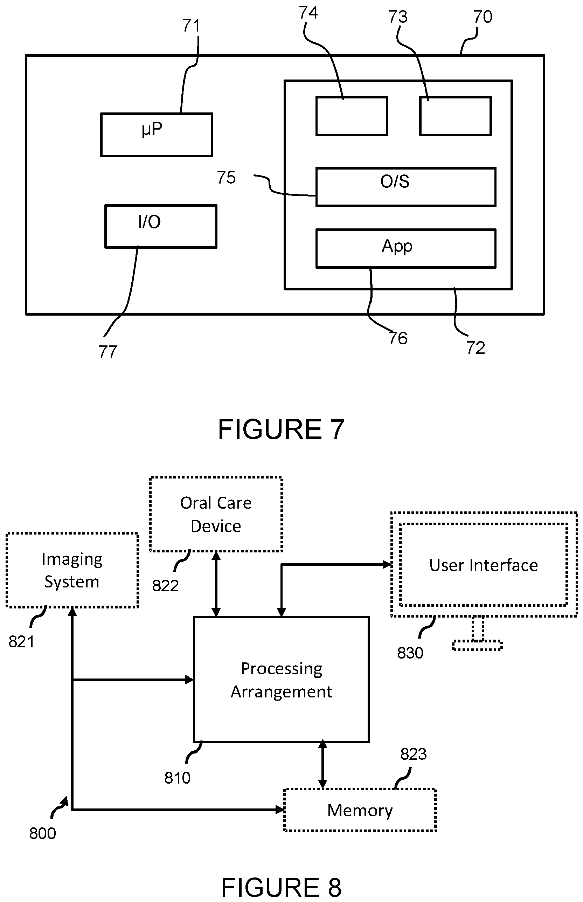

By way of further example, Figure 7 illustrates an example of a processing arrangement 70 within which one or more parts of an embodiment may be employed. Various operations discussed above may utilize the capabilities of the processing arrangement 70. For example, one or more parts of a system for generating one or more recommended cleaning and/or treatment characteristics may be incorporated in any element, module, application, and/or component discussed herein. In this regard, it is to be understood that system functional blocks can run on a single computer or may be distributed over several computers and locations (e.g. connected via internet).

The processing arrangement 70 includes, but is not limited to, PCs, workstations, laptops, PDAs, palm devices, servers, storages, and the like. Generally, in terms of hardware architecture, the processing arrangement 70 may include one or more processors 71, memory 72, and one or more I/O devices 77 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 71 is a hardware device for executing software that can be stored in the memory 72. The processor 71 can be virtually any custom made or commercially available processor, a central processing unit (CPU), a digital signal processor (DSP), or an auxiliary processor among several processors associated with the processing arrangement 70, and the processor 71 may be a semiconductor based microprocessor (in the form of a microchip) or a microprocessor.

The memory 72 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 72 may incorporate electronic, magnetic, optical, and/or other types of storage media. Note that the memory 72 can have a distributed architecture, where various components are situated remote from one another, but can be accessed by the processor 71.

The software in the memory 72 may include one or more separate programs, each of which comprises an ordered listing of executable instructions for implementing logical functions. The software in the memory 72 includes a suitable operating system (O/S) 75, compiler 74, source code 73, and one or more applications 76 in accordance with exemplary embodiments. As illustrated, the application 76 comprises numerous functional components for implementing the features and operations of the exemplary embodiments. The application 76 of the processing arrangement 70 may represent various applications, computational units, logic, functional units, processes, operations, virtual entities, and/or modules in accordance with exemplary embodiments, but the application 76 is not meant to be a limitation.

The operating system 75 controls the execution of other computer programs, and provides scheduling, input-output control, file and data management, memory management, and communication control and related services. It is contemplated by the inventors that the application 76 for implementing exemplary embodiments may be applicable on all commercially available operating systems.

Application 76 may be a source program, executable program (object code), script, or any other entity comprising a set of instructions to be performed. When a source program, then the program is usually translated via a compiler (such as the compiler 74), assembler, interpreter, or the like, which may or may not be included within the memory 72, so as to operate properly in connection with the O/S 75. Furthermore, the application 76 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 77 may include input devices such as, for example but not limited to, a mouse, keyboard, scanner, microphone, camera, etc. Furthermore, the I/O devices 77 may also include output devices, for example but not limited to a printer, display, etc. Finally, the I/O devices 77 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 77 also include components for communicating over various networks, such as the Internet or intranet.

If the processing arrangement 70 is a PC, workstation, intelligent device or the like, the software in the memory 72 may further include a basic input output system (BIOS) (omitted for simplicity). The BIOS is a set of essential software routines that initialize and test hardware at startup, start the O/S 75, and support the transfer of data among the hardware devices. The BIOS is stored in some type of read-only-memory, such as ROM, PROM, EPROM, EEPROM or the like, so that the BIOS can be executed when the processing arrangement 70 is activated.

When the processing arrangement 70 is in operation, the processor 71 is configured to execute software stored within the memory 72, to communicate data to and from the memory 72, and to generally control operations of the processing arrangement 70 pursuant to the software. The application 76 and the O/S 75 are read, in whole or in part, by the processor 71, perhaps buffered within the processor 71, and then executed.

When the application 76 is implemented in software it should be noted that the application 76 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 76 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

Figure 8 illustrates a processing system 800 according to an embodiment.

The processing system 800 comprises a processing arrangement 810 according to an embodiment. The processing arrangement is configured to carry out a method previously herein described, and may be embodied as previously described with reference to Figure 7.

In particular, the processing arrangement may be configured to obtain, for each of a plurality of first parts of the mouth of the subject, quality data indicating a quality of said first part of the mouth; and generate, for each of two or more second parts of the mouth of the subject, one or more recommended cleaning and/or treatment characteristics for said second part of the mouth by processing the quality data of any first parts of the mouth overlapping said second part of the mouth of the subject.

The processing arrangement 810 may, for instance, obtain the quality data by communicating with an imaging system 821 (e.g. by processing image data provided by the imaging system) and/or a memory (e.g. storing the quality data generated elsewhere) and/or the user interface 830 (configured to receive a user input that controls or defines the quality data).

The processing arrangement 810 may control a user interface 830 to provide a user-perceptible output representing the recommended cleaning and/or treatment characteristics. The user interface may form part of the processing system 800. The user interface may, for instance, be an application of a smartphone or other smart device, or a dashboard in a dental office/clinic, or a medical control center.

Of course, the processing arrangement may be adapted to store the recommended cleaning and/or treatment characteristics in the memory 823.

The processing system may comprise the oral care device 822, configured to perform cleaning and/or treatment on the mouth of the subject, e.g. a toothbrush, an oral irrigator, a flosser, a tooth whitening device and so on. The processing arrangement 810 may be configured to obtain sensor data from one or more sensors on the oral care device to facilitate monitoring and/or dynamically adapting of actual and recommended cleaning and/or treatment characteristics during a cleaning and/or treatment procedure.

The processing arrangement 810 may form part of any suitable processor, e.g. an application of a smartphone or smart device or integrated into the oral care device and/or imaging system.

The skilled person would be readily capable of developing a processing system for carrying out any herein described method. Thus, each step of the flow chart may represent a different action performed by a processing system and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of a computer program comprising code means for implementing any described method when said program is run on a processing system, such as a computer. Thus, different portions, lines, or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A computer-implemented method of generating recommended cleaning and/or treatment characteristics for different parts of a mouth of a subject, the computer-implemented method comprising:
obtaining, for each of a plurality of first parts of the mouth of the subject, quality data indicating a quality of said first part of the mouth; and
generating, for each of two or more second parts of the mouth of the subject, one or more recommended cleaning and/or treatment characteristics for said second part of the mouth by processing the quality data of any first parts of the mouth overlapping said second part of the mouth of the subject.

2. The computer-implemented method of claim 1, wherein, for each of two or more second parts of the mouth of the subject, the step of generating one or more recommended cleaning and/or treatment characteristics comprises generating a recommended cleaning and/or treatment time.

3. The computer-implemented method of claim 2, wherein the step of generating a recommended cleaning and/or treatment time for each second part of the mouth of the subject comprises:
obtaining a desired total cleaning and/or treatment time;
distributing the desired total cleaning and/or treatment time between each second part of the mouth of the subject, based on the assessed quality of any first parts the mouth overlapping said second part of the mouth of the subject.

4. The computer-implemented method of claim 2, wherein the step of generating a recommended cleaning and/or treatment time for each second part of the mouth of the subject comprises determining a recommended cleaning and/or treatment time for each second part of the mouth of the subject, based on the assessed quality of any first parts the mouth overlapping said second part of the mouth of the subject, independently of any other recommended cleaning and/or treatment times for any other second part of the mouth of the subject.

5. The computer-implemented method of any of claims 1 to 4, wherein each first part of the mouth of the subject is an area of the mouth of the subject that should contain only a single oral surface or only a single tooth.

6. The computer-implemented method of any of claims 1 to 5, wherein each second part of the mouth of the subject represents:
a different segment or quarter of the mouth of the subject; or
a different tooth of the mouth of the subject.

7. The computer-implemented method of any of claims 1 to 6, wherein:
the quality data of each two or more first parts of the mouth of the subject comprises a ranking of said first part of the mouth of the subject, wherein the ranking is based on a health or disease status of the said first part of the mouth of the subject; and
the step of generating the one or more recommended cleaning and/or treatment characteristics for each second part of the mouth of the subject is responsive to the rankings of any first parts of the mouth overlapping said second part of the mouth of the subject.

8. The computer-implemented method of any of claims 1 to 7, wherein:
the quality data of each two or more first parts of the mouth of the subject comprises an identification of any missing teeth and/or additional teeth in each first part of the mouth of the subject; and
the step of generating the one or more recommended cleaning and/or treatment characteristics for each second part of the mouth is responsive to the presence or absence of any missing teeth and/or additional teeth in any first parts of the mouth of the subject overlapping said second part of the mouth of the subject.

9. The computer-implemented method of any of claims 1 to 8, wherein:
the quality data of each two or more first parts of the mouth of the subject comprises an identification of any teeth with any restorations, in each first part of the mouth of the subject; and
the step of generating one or more recommended cleaning and/or treatment characteristics for each second part of the mouth is responsive to the presence or absence of any teeth with restorations in any first parts of the mouth of the subject overlapping said second part of the mouth of the subject.

10. The computer-implemented method of any of claims 1 to 9, wherein:
the quality data of two or more first parts of the mouth of the subject comprises a classification of a risk, health status and/or disease status of each first part of the mouth of the subject; and
the step of generating one or more recommended cleaning and/or treatment characteristics for each second part of the mouth is responsive to any classifications of any first parts of the mouth of the subject overlapping said second part of the mouth of the subject.

11. The computer-implemented method of any of claims 1 to 10, wherein the size of each first part of the mouth is smaller than or equal to the size of any of the second parts of the mouth.

12. The computer-implemented method of any of claims 1 to 11, further comprising generating a first subject-perceptible output representing the one or more recommended cleaning and/or treatment characteristics for each second part of the mouth of the subject.

13. A computer-implemented method of monitoring a cleaning and/or treatment of a mouth of a subject, the computer-implemented method comprising:
obtaining the one or more recommended cleaning and/or treatment characteristics for different parts of the mouth of the subject generated by the method of any of claims 1 to 12, wherein the one or more recommended cleaning and/or treatment characteristics includes a cleaning and/or treatment time;
monitoring a cleaning and/or treatment of the second parts of the mouth of the subject; and
for each second part of the mouth of the subject, controlling a second subject-perceptible output based on whether or not a cleaning and/or treatment of said second part of the mouth of the subject exceeds a recommended cleaning and/or treatment time.

14. A computer program product comprising computer program code means which, when executed on a computing device having a processing system, cause the processing system to perform all the steps of the method according to any of claims 1 to 13.

15. A processing arrangement configured to generate recommended cleaning and/or treatment characteristics for different regions of a mouth of a subject, the processing arrangement being configured to:
obtain, for each of a plurality of first parts of the mouth of the subject, quality data indicating a quality of said first part of the mouth; and
generate, for each of two or more second parts of the mouth of the subject, one or more recommended cleaning and/or treatment characteristics for said second part of the mouth by processing the quality data of any first parts of the mouth overlapping said second part of the mouth of the subject.
